(19) Europäisches Patentamt European Patent Office Office européen des brevets

(11) **EP 3 936 549 A1**

(12) **EUROPEAN PATENT APPLICATION**
published in accordance with Art. 153(4) EPC

(43) Date of publication:
**12.01.2022 Bulletin 2022/02**

(51) Int Cl.:
**C08G 59/42** (2006.01)     **C08L 101/14** (2006.01)
**A61F 13/53** (2006.01)

(21) Application number: **20770016.2**

(86) International application number:
**PCT/JP2020/009468**

(22) Date of filing: **05.03.2020**

(87) International publication number:
**WO 2020/184386 (17.09.2020 Gazette 2020/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.03.2019 JP 2019042303**

(71) Applicant: **Sumitomo Seika Chemicals Co., Ltd.**
**Kako-gun, Hyogo 675-0145 (JP)**

(72) Inventor: **KAWAHARA, Toru**
**Himeji-shi, Hyogo 672-8076 (JP)**

(74) Representative: **Nederlandsch Octrooibureau**
**P.O. Box 29720**
**2502 LS The Hague (NL)**

(54) **WATER ABSORBING RESIN PARTICLES, ABSORBENT ARTICLE, METHOD FOR MANUFACTURING WATER ABSORBING RESIN PARTICLES, METHOD FOR FACILITATING PERMEATION OF PHYSIOLOGICAL SALINE SOLUTION INTO ABSORBENT BODY**

(57)     Water-absorbent resin particles in which a gel outflow proportion is 0% to 20% are disclosed. The gel outflow proportion is measured by a method including: measuring a mass W (g) of 20 g of a swollen gel flowed out from a through hole of a disc while the swollen gel is compressed by a weight of 2000 g for 30 seconds, where the swollen gel is formed by allowing the water-absorbent resin particles to absorb physiological saline; and calculating the gel outflow proportion (%) by the following formula.

$$\text{Gel outflow proportion (\%)} = (W/20) \times 100$$

**Fig.2**

**Description**

**Technical Field**

The present invention relates to water-absorbent resin particles, an absorbent article, a method for producing water-absorbent resin particles, and a method for increasing a permeation rate of physiological saline into an absorber after the absorber is applied with a load in a state where the absorber has absorbed water.

**Background Art**

In the related art, an absorber containing water-absorbent resin particles has been used in an absorbent article for absorbing a liquid having water as a main component such as urine. For example, Patent Literature 1 discloses a water-absorbent resin having an excellent water-absorbent magnification in a non-pressurization state, an excellent water-absorbent characteristics under a high load, and a small amount of residual monomers.

**Citation List**

**Patent Literature**

[Patent Literature 1] Japanese Unexamined Patent Publication No. 2006-199805

**Summary of Invention**

**Technical Problem**

A load may be applied to an absorbent article having an absorber in a state of absorbing water. It is desirable that the absorber can absorb urine and the like at a high permeation rate even after a load has been applied in a state where the absorber absorbed water.
One aspect of the present invention provides water-absorbent resin particles capable of increasing a permeation rate of physiological saline into an absorber after the absorber is applied with a load in a state where the absorber has absorbed water.

**Solution to Problem**

One aspect of the present invention relates to water-absorbent resin particles in which a gel outflow proportion measured by a method including the following (1), (2), (3), (4), (5), (6), and (7) in this order in an environment of 25°C ± 2°C is 0% to 20%:

(1) fixing a tube shaped body of circular cross section with an inner diameter of 5.0 cm having a bottom part which blocks one end portion of the tube shaped body, the bottom part forming an opening with a diameter of 3.0 cm at a central portion, in a direction so that a longitudinal direction of the tube shaped body is vertical and the bottom part is at a downward side;
(2) disposing a disc with a diameter of 4.9 cm and a thickness of 3.0 mm on the bottom part in an inner side of the tube shaped body in a direction so that a main surface of the disc is horizontal, the disc forming a through hole at a central portion, the through hole having a wall surface inclined with respect to the main surface of the disc so that the through hole is narrowest at a central location in a thickness direction of the disc, a diameter of the through hole being 8.0 mm as a maximum value at locations of both main surfaces of the disc and being 5.0 mm as a minimum value at the central location in the thickness direction of the disc;
(3) putting 20 g of a swollen gel into the tube shaped body, thereby disposing the swollen gel on the disc, the swollen gel being formed by allowing the water-absorbent resin particles to absorb physiological saline, the swollen gel having a mass 30 times the mass of the water-absorbent resin particles before absorbing the physiological saline;
(4) compressing the swollen gel for 30 seconds by placing a column shaped first weight with a diameter of 4.9 cm on the swollen gel in the tube shaped body, the first weight having a mass of 500 g;
(5) further compressing the swollen gel under a load of 2000 g by placing a column shaped second weight with a diameter of 4.9 cm on the first weight, the second weight having a mass of 1500 g;
(6) measuring a mass W (g) of the swollen gel flowed out from the through hole of the disc while the swollen gel is compressed for 30 seconds under a load of 2000 g; and
(7) calculating the gel outflow proportion (%) by the following formula,

$$gel\ outflow\ proportion\ (\%) = (W/20) \times 100.$$

A small gel outflow proportion means that it is difficult for a swollen gel, which is formed when the water-absorbent resin particles absorb a large amount of physiological saline, to move in a state where a load is applied. When an absorber absorbs a liquid, a swollen gel is formed inside. It is thought that, if the movement of the swollen gel is small when a load is applied to the absorber, the swollen gel in the portion to which the load is applied remains unmoved so that physiological saline can be absorbed even when the physiological saline is added again, and therefore a permeation rate remains high. On the other hand, it is thought that, if the movement of the swollen gel is large when a load is applied, the swollen gel in the portion to which the load is applied moves in an outward direction of the loaded portion so that an absorbable amount is limited in the loaded portion when physiological saline is added again, and therefore a permeation rate is lowered.

Another aspect of the present invention relates to an absorbent article including: a liquid impermeable sheet; an absorber; and a liquid permeable sheet, in which the liquid impermeable sheet, the absorber, and the liquid permeable sheet are disposed in this order. The absorber contains the above-described water-absorbent resin particles.

Still another aspect of the present invention relates to a method for producing water-absorbent resin particles, the method including sorting out water-absorbent resin particles in which the above-mentioned gel outflow proportion is 0% to 20%.

Still another aspect of the present invention relates to a method for increasing a permeation rate of physiological saline into an absorber which contains water-absorbent resin particles after the absorber is applied with a load in a state where the absorber has absorbed water. This method includes reducing a gel outflow proportion of the water-absorbent resin particles.

**Advantageous Effects of Invention**

According to one aspect of the present invention, water-absorbent resin particles, which are capable of increasing a permeation rate of physiological saline into an absorber after the absorber is applied with a load in a state where the absorber has absorbed water, are provided.

**Brief Description of Drawings**

Fig. 1 is a schematic view showing a method for measuring a gel outflow proportion of water-absorbent resin particles.
Fig. 2 is a schematic view showing the method for measuring a gel outflow proportion of water-absorbent resin particles.
Fig. 3 is a schematic view showing the method for measuring a gel outflow proportion of water-absorbent resin particles.
Fig. 4 is a cross-sectional view showing one embodiment of an absorbent article.
Fig. 5 is a plan view showing an example of a stirring blade.
Fig. 6 is a schematic view showing a method for measuring a water absorption amount of water-absorbent resin particles for physiological saline under a load of 4.14 kPa.

**Description of Embodiments**

Hereinafter, some embodiments of the present invention will be described in detail. However, the present invention is not limited to the following embodiments.

In the present specification, "acrylic" and "methacrylic" are collectively referred to as "(meth)acrylic." "Acrylate" and "methacrylate" are also referred to as "(meth)acrylate." "(Poly)" means both of a case where there is a prefix of "poly" and a case where there is no prefix thereof. Regarding numerical value ranges described in a stepwise manner in the present specification, an upper limit value or a lower limit value of a numerical value range in a certain step can be arbitrarily combined with an upper limit value or a lower limit value of a numerical value range in another step. In a numerical value range described in the present specification, an upper limit value or a lower limit value of the numerical value range may be replaced with a value shown in examples. "A or B" means one of A and B, or both of A and B. The term "water-soluble" means that a solubility of 5% by mass or more is exhibited in water at 25°C. For materials exemplified in the present specification, one kind may be used alone, or two or more kinds may be used in combination. In a case where there are a plurality of substances corresponding to each of components in a composition, a content of each of the components in the composition means a total amount of the plurality of substances present in the composition unless otherwise specified.

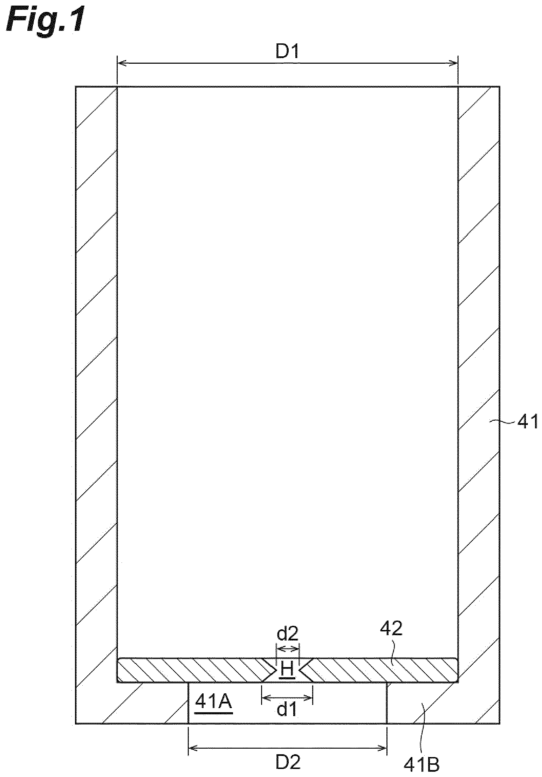

Water-absorbent resin particles according to the present embodiment show a gel outflow proportion of 0% to 20%. Fig. 1, Fig. 2, and Fig. 3 are schematic views showing a method for measuring the gel outflow proportion of the water-absorbent resin particles. The method shown in Figs. 1 to 3 includes the following steps (1), (2), (3), (4), (5), (6), and (7) in this order. The gel outflow proportion is measured in an environment of 25°C ± 2°C.

Evaluation of gel outflow proportion

(1) A tube shaped body 41 with a circular cross section and an inner diameter of D1 (= 5.0 cm) having a bottom part 41B which blocks one end portion of the tube shaped body 41 is fixed in a direction so that a longitudinal direction of the tube shaped body 41 is vertical and the bottom part 41B is at a downward side (Fig. 1). The bottom part 41B forms an opening 41A with a diameter of D2 (= 3.0 cm) at a central portion.

(2) A disc 42 with a diameter of 4.9 cm and a thickness of 3.0 mm is disposed on the bottom part 41B in an inner side of the tube shaped body 41a in a direction so that a main surface of the disc 42 is horizontal (Fig. 1). The disc 42 forms a through hole H at a central portion. The through hole H has a wall surface inclined with respect to the main surface of the disc 42 so that the through hole H is narrowest at a central location in a thickness direction of the disc 42. A diameter of the through hole H is a maximum value d1 (= 8.0 mm) at locations of both main surfaces of the disc 42 and is a minimum value d2 (= 5.0 mm) at the central location in the thickness direction of the disc 42.

(3) 20 g of a swollen gel 45 which is formed by allowing the water-absorbent resin particles to absorb physiological saline is put into the tube shaped body 41 and thereby disposed on the disc 42. The swollen gel 45 has a mass 30 times the mass of the water-absorbent resin particles before absorbing the physiological saline.

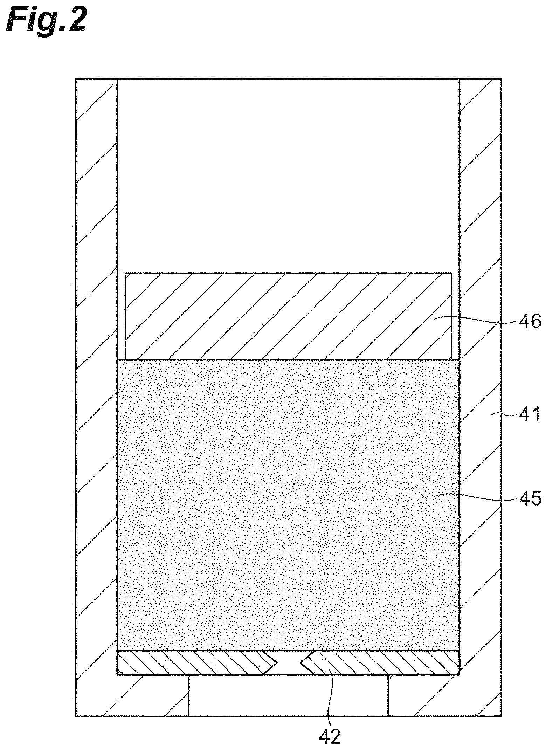

(4) The swollen gel 45 is compressed for 30 seconds by placing a column shaped first weight 46 with a diameter of 4.9 mm having a mass of 500 g on the swollen gel 45 in the tube shaped body 41 (Fig. 2).

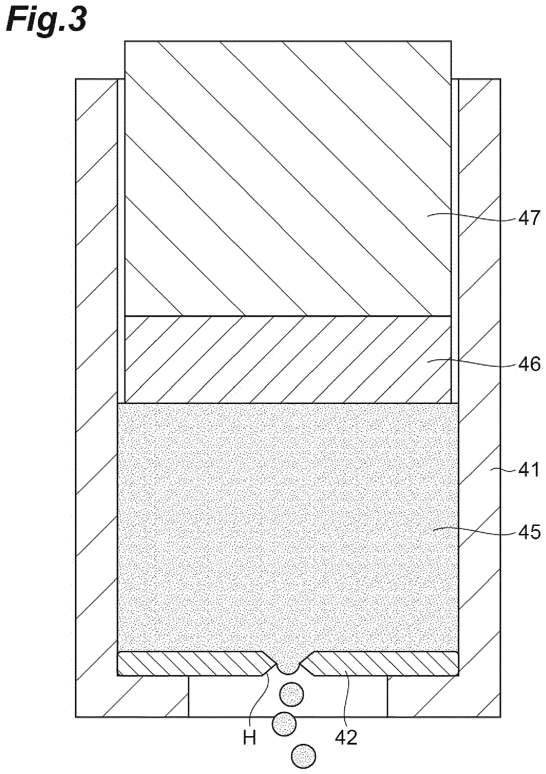

(5) The swollen gel 45 is further compressed under a load of 2000 g by placing a column shaped second weight 47 with a diameter of 4.9 mm having a mass of 1500 g on the first weight 46 (Fig. 3).

(6) A mass W (g) of the swollen gel 45 flowed out from the through hole H of the disc 42 while the swollen gel 45 is compressed for 30 seconds under a load of 2,000 g, is measured.

(7) The gel outflow proportion (%) is calculated by the following formula.

$$\text{Gel outflow proportion }(\%) = (W/20) \times 100$$

The tube shaped body 41 may be, for example, an acrylic resin molded body. The tube shaped body 41 is fixed with a fixing instrument such as a clamp. Each of the weights is gently placed on the swollen gel 45 so that a momentary strong impact due to dropping is not applied to the swollen gel 45. The entire amount of the swollen gel 45 flowed out from the through hole H within 30 seconds from the start of the application of a load of 2000 g by the first weight 46 and the second weight 47 is collected, and a mass W (g) thereof is measured. Flowed out portions of the swollen gel 45 are often dropped, but in a case where a part or all of the flowed out portions of the swollen gel 45 are held in a state of being hung down from the through hole H, a mass W of the flowed out portions of the swollen gel 45, including the portions of the swollen gel which are hung down at a location below the lower side main surface of the disc 42, is measured.

The gel outflow proportion of the water-absorbent resin particles may be 15% or less, 10% or less, or 5% or less. In a case where the swollen gel 45 is not flowed out from the through hole H at all, the gel outflow proportion is 0%, which is the lower limit of the gel outflow proportion.

A water absorption amount of the water-absorbent resin particles for physiological saline under a load of 4.14 kPa (hereinafter, may be referred to as a "water absorption amount under a load") may be 12 mL/g or more. When the water absorption amount of the water-absorbent resin particles under a load is 12 mL/g, the gel outflow proportion tends to become small. Thereby, it is possible to further increase a permeation rate of physiological saline into an absorber to which a load has been applied in a state where the absorber absorbed water. Furthermore, when a water absorption amount under a load is large, this is also advantageous from the viewpoint that gel blocking is then unlikely to occur in an absorbent article. The water absorption amount of the water-absorbent resin particles under a load may be 14 mL/g or more, 16 mL/g or more, 18 mL/g or more, or 20 mL/g or more, and may be 35 mL/g or less, 30 mL/g or less, 28 mL/g or less, or 26 mL/g or less. The water absorption amount under a load is measured by a method described in Examples to be described later.

The water retention amount of the water-absorbent resin particles for physiological saline may be 35 g/g or more, 36 g/g or more, 37 g/g or more, 38 g/g or more, 39 g/g or more, or 40 g/g or more. The water retention amount of the water-absorbent resin particles for physiological saline may be 50 g/g or less, 49 g/g or less, 48 g/g or less, 47 g/g or less, 46 g/g or less, or 45 g/g or less. Furthermore, the water retention amount of the water-absorbent resin particles for physiological saline may be 35 to 50 g/g, 38 to 48 g/g, or 40 to 46 g/g. When the water retention amount is within these ranges, a permeation rate in an absorber tends to become high, and liquid leakage from the absorber tends to be inhibited. The water absorption amount of the water-absorbent resin particles for physiological saline is measured by a method described in Examples to be described later.

Examples of shapes of the water-absorbent resin particles according to the present embodiment include a substantially spherical shape, a crushed shape, a granular shape, and the like. A median particle size of the water-absorbent resin particles according to the present embodiment may be 250 μm or more and 850 μm or less, 700 μm or less, or 600 μm or less, or may be 300 μm or more and 850 μm or less, 700 μm or less, or 600 μm or less. The water-absorbent resin particles according to the present embodiment may have a desired particle size distribution at a timing obtained in a production method to be described later, but their particle size distribution may be adjusted by performing operations such as adjustment of a particle size through classification with a sieve.

The water-absorbent resin particles according to the present embodiment can contain, for example, a crosslinking polymer formed by polymerization of monomers including ethylenically unsaturated monomers. The crosslinking polymer has a monomer unit derived from an ethylenically unsaturated monomer.

The water-absorbent resin particles can be produced by a method including a step of polymerizing monomers including ethylenically unsaturated monomers. Examples of methods of polymerization include a reverse phase suspension polymerization method, an aqueous solution polymerization method, a bulk polymerization method, and a precipitation polymerization method. The polymerization method may be the reverse phase suspension polymerization method or the aqueous solution polymerization method from the viewpoints of facilitating securement of favorable water-absorbent characteristics of the obtained water-absorbent resin particles and control of a polymerization reaction. Hereinbelow, a method for polymerizing ethylenically unsaturated monomers will be described with the reverse phase suspension polymerization method as an example.

An ethylenically unsaturated monomer may be water-soluble. Examples of water-soluble ethylenically unsaturated monomers include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where an ethylenically unsaturated monomer has an amino group, the amino group may be quaternarized. The ethylenically unsaturated monomer may be used alone or in a combination of two or more kinds thereof. A functional group, such as a carboxyl group and an amino group, of the monomer, may function as a crosslinkable functional group in a surface crosslinking step to be described later.

From the viewpoint of high industrial availability, the ethylenically unsaturated monomer may include at least one compound selected from the group consisting of (meth)acrylic acid and a salt thereof, acrylamide, methacrylamide, and N,N-dimethyl acrylamide, or may include at least one compound selected from the group consisting of (meth)acrylic acid and a salt thereof and acrylamide. The ethylenically unsaturated monomer may include at least one compound selected from the group consisting of (meth)acrylic acid and a salt thereof from the viewpoint of further enhancing water-absorbent characteristics.

Usually, the ethylenically unsaturated monomers are suitably used in a form of an aqueous solution. A concentration of the ethylenically unsaturated monomers in an aqueous solution containing the ethylenically unsaturated monomers (hereinafter, simply referred to as a "monomer aqueous solution") may be 20% by mass or more and a saturated concentration or less, 25% to 70% by mass, or 30% to 55% by mass. Examples of water to be used in an aqueous solution include tap water, distilled water, and ion-exchanged water.

For the monomer for obtaining the water-absorbent resin particles, a monomer other than the above-described ethylenically unsaturated monomers may be used. Such a monomer can be used by, for example, being mixed with an aqueous solution containing the above-described ethylenically unsaturated monomers. A use amount of the ethylenically unsaturated monomers may be 70 to 100 mol% with respect to a total amount of monomers. A proportion of (meth)acrylic acid and a salt thereof may be 70 to 100 mol% with respect to a total amount of monomers.

In a case where ethylenically unsaturated monomers have an acid group, a monomer aqueous solution may be used after neutralizing this acid group with an alkaline neutralizing agent. From the viewpoint of increasing an osmotic pressure of the obtained water-absorbent resin particles and thereby further enhancing water-absorbent characteristics (such as a water absorption amount), a degree of neutralization in the ethylenically unsaturated monomers by the alkaline neutralizing agent may be 10 to 100 mol%, 50 to 90 mol%, or 60 to 80 mol% of the acid group in the ethylenically unsaturated monomers. Examples of alkaline neutralizing agents include alkali metal salts such as sodium hydroxide, sodium carbonate, sodium hydrogen carbonate, potassium hydroxide, and potassium carbonate; ammonia; and the like. The alkaline neutralizing agent may be used alone or in combination of two or more kinds thereof. The alkaline neutralizing agent may be used in a form of an aqueous solution to simplify a neutralizing operation. Neutralization of the acid groups in the ethylenically unsaturated monomers can be performed by, for example, adding an aqueous solution of sodium hydroxide, potassium hydroxide, or the like dropwise to the above-described monomer aqueous solution and mixing them.

In the reverse phase suspension polymerization method, a monomer aqueous solution can be dispersed in a hydrocarbon dispersion medium in the presence of a surfactant, and polymerization of ethylenically unsaturated monomers can be performed using a radical polymerization initiator or the like. As the radical polymerization initiator, it is possible to use a water-soluble radical polymerization initiator.

Examples of surfactants include nonionic surfactants and anionic surfactants. Examples of nonionic surfactants include sorbitan fatty acid esters, polyglycerin fatty acid esters, sucrose fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene hydrogenated castor oil, alkylallyl formaldehyde condensed polyoxyethylene ethers, polyoxyethylene polyoxypropylene block copolymers, polyoxyethylene polyoxypropyl alkyl ethers, and polyethylene glycol fatty acid esters. Examples of anionic surfactants include fatty acid salts, alkylbenzene sulfonate, alkylmethyl taurate, polyoxyethylene alkylphenyl ether sulfuric acid ester salts, polyoxyethylene alkyl ether sulfonic acid salts, phosphoric acid esters of polyoxyethylene alkyl ethers, and phosphoric acid esters of polyoxyethylene alkyl allyl ethers. The surfactant may be used alone or in combination of two or more kinds thereof.

The surfactant may include at least one compound selected from the group consisting of sorbitan fatty acid esters, polyglycerin fatty acid esters, and sucrose fatty acid esters, from the viewpoints that then, a state of a W/O type reverse phase suspension becomes favorable, water-absorbent resin particles having a suitable particle size are easily obtained, and industrial availability becomes high. The surfactant may include sucrose fatty acid esters or may include sucrose stearic acid esters from the viewpoint that water-absorbent characteristics of the obtained water-absorbent resin particles are then easily improved.

A use amount of the surfactant may be 0.05 to 10 parts by mass, 0.08 to 5 parts by mass, or 0.1 to 3 parts by mass, with respect to 100 parts by mass of the monomer aqueous solution, from the viewpoint that a sufficient effect is obtained within these use amounts, and these amounts are economic.

In the reverse phase suspension polymerization, a polymer-based dispersant may be used in combination with the above-mentioned surfactant. Examples of polymer-based dispersants include maleic anhydride-modified polyethylene, maleic anhydride-modified polypropylene, a maleic anhydride-modified ethylene-propylene copolymer, a maleic anhydride-modified EPDM (ethylene propylene diene terpolymer), maleic anhydride-modified polybutadiene, a maleic anhydride-ethylene copolymer, a maleic anhydride-propylene copolymer, a maleic anhydride-ethylene-propylene copolymer, a maleic anhydride-butadiene copolymer, polyethylene, polypropylene, an ethylene-propylene copolymer, oxidized polyethylene, oxidized polypropylene, an oxidized ethylene-propylene copolymer, an ethylene-acrylic acid copolymer, ethyl cellulose, ethyl hydroxyethyl cellulose, and the like. The polymer-based dispersant may be used alone or in combination of two or more kinds thereof. From the viewpoint of excellent dispersion stability of monomers, the polymer-based dispersant may include at least one selected from the group consisting of maleic anhydride-modified polyethylene, maleic anhydride-modified polypropylene, a maleic anhydride-modified ethylene-propylene copolymer, a maleic anhydride-ethylene copolymer, a maleic anhydride-propylene copolymer, a maleic anhydride-ethylene-propylene copolymer, polyethylene, polypropylene, an ethylene-propylene copolymer, oxidized polyethylene, oxidized polypropylene, and an oxidized ethylene-propylene copolymer.

A use amount of the polymer-based dispersant may be 0.05 to 10 parts by mass, 0.08 to 5 parts by mass, or 0.1 to 3 parts by mass, with respect to 100 parts by mass of the monomer aqueous solution, from the viewpoint that a sufficient effect is obtained within these use amounts, and these amounts are economic.

The hydrocarbon dispersion medium may include at least one compound selected from the group consisting of a chained aliphatic hydrocarbon having 6 to 8 carbon atoms and an alicyclic hydrocarbon having 6 to 8 carbon atoms. Examples of hydrocarbon dispersion media include chained aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. The hydrocarbon dispersion medium may be used alone or in combination of two or more kinds thereof.

The hydrocarbon dispersion medium may include at least one selected from the group consisting of n-heptane and cyclohexane from the viewpoints of high industrial availability and stable qualities. From the same viewpoints, as a mixture of the hydrocarbon dispersion media, for example, a commercially available Exxsol Heptane (manufactured by ExxonMobil Chemical: containing n-heptane and 75% to 85% of hydrocarbons of isomers thereof) may be used.

A use amount of the hydrocarbon dispersion medium may be 30 to 1,000 parts by mass, 40 to 500 parts by mass, or 50 to 300 parts by mass, with respect to 100 parts by mass of the monomer aqueous solution, from the viewpoint that polymerization heat is then appropriately removed, and thereby a polymerization temperature is easily controlled. In a case where a use amount of the hydrocarbon dispersion medium is 30 parts by mass or more, there is a tendency that it becomes easy to control a polymerization temperature. In a case where a use amount of the hydrocarbon dispersion medium is 1,000 parts by mass or less, productivity of polymerization tends to be improved, which is economical.

A radical polymerization initiator may be water-soluble. Examples of water-soluble radical polymerization initiators include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-amidinopropane) dihydrochloride, 2,2'-azobis[2-(N-phenylamidino)propane] dihydrochloride, 2,2'-azo-bis[2-(N-allylamidino)propane] dihydrochloride, 2,2'-azobis[2-(2-imidazolin-2-yl)propane] dihydrochloride, 2,2'-azo-bis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). The radical polymerization initiator may be used alone or in combination of two or more kinds thereof. The radical polymerization initiator may be at least one selected from the group consisting of potassium persulfate, ammonium persulfate, sodium persulfate, 2,2'-azobis(2-amidinopropane) dihydrochloride, 2,2'-azobis[2-(2-imidazolin-2-yl)propane] dihydrochloride, and 2,2'-azobis{2-[1-(2-hydroxyethyl)-2-imidazolin-2-yl]propane} dihydrochloride.

A use amount of the radical polymerization initiator may be 0.00005 to 0.01 moles with respect to 1 mole of the ethylenically unsaturated monomers. A case in which a use amount of the radical polymerization initiator is 0.00005 moles or more is efficient, because then a polymerization reaction is not required to be performed for a long period of time. In a case where a use amount of the radical polymerization initiator is 0.01 moles or less, it is easy to inhibit occurrence of a rapid polymerization reaction.

The radical polymerization initiator can also be used as a redox polymerization initiator when it is used in combination with a reducing agent such as sodium sulfite, sodium hydrogen sulfite, ferrous sulfate, and L-ascorbic acid.

In a polymerization reaction, a monomer aqueous solution used for the polymerization may contain a chain transfer agent. Examples of chain transfer agents include hypophosphites, thiols, thiolic acids, secondary alcohols, and amines.

The monomer aqueous solution used for the polymerization may contain a thickener to control a particle size of the water-absorbent resin particles. Examples of thickeners include hydroxyethyl cellulose, hydroxypropyl cellulose, methyl cellulose, carboxymethyl cellulose, polyacrylic acid, polyethylene glycol, polyacrylamide, polyethyleneimine, dextrin, sodium alginate, polyvinyl alcohol, polyvinylpyrrolidone, polyethylene oxide, and the like. In a case where stirring speeds in the polymerization are the same, a median particle size of particles to be obtained is likely to become large as a viscosity of the monomer aqueous solution becomes high.

Crosslinking by self-crosslinking may occur during polymerization, but crosslinking may be further performed by using an internal crosslinking agent. By using the internal crosslinking agent, it is easy to control water-absorbent characteristics of the water-absorbent resin particles. The internal crosslinking agent is generally added to a reaction solution in the polymerization reaction. Examples of internal crosslinking agents include di- or tri(meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the polyols with unsaturated acids (such as maleic acid and fumaric acid); bis(meth)acrylamides such as N,N'-methylenebis(meth)acrylamide; di- or tri(meth)acrylic acid esters obtained by reacting a polyepoxide with (meth)acrylic acid; carbamyl di(meth)acrylate esters obtained by reacting a polyisocyanate (such as tolylene diisocyanate and hexamethylene diisocyanate) with hydroxyethyl (meth)acrylate; compounds having two or more polymerizable unsaturated groups, such as allylated starch, allylated cellulose, diallyl phthalate, N,N',N"-triallylisocyanate, and divinylbenzene; polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromohydrin, and $\alpha$-methyl epichlorohydrin; compounds having two or more reactive functional groups, such as isocyanate compounds (such as 2,4-tolylene diisocyanate and hexamethylene diisocyanate); and the like. The internal crosslinking agent may be used alone or in combination of two or more kinds thereof. The internal crosslinking agent may include a polyglycidyl compound or a diglycidyl ether compound, or may include at least one selected from the group consisting of (poly)ethylene glycol diglycidyl ether, (poly)propylene glycol diglycidyl ether, and (poly)glycerin diglycidyl ether.

A use amount of the internal crosslinking agent may be 0 mmol or more, 0.02 mmol or more, 0.03 mmol or more, 0.04 mmol or more, or 0.05 mmol or more, and may be 0.1 moles or less, per 1 mole of the ethylenically unsaturated monomer, from the viewpoints of inhibiting water-soluble properties by appropriately crosslinking the obtained polymer, and easily obtaining a sufficient water absorption amount.

An aqueous phase containing an ethylenically unsaturated monomer, a radical polymerization initiator, and if necessary, an internal crosslinking agent, and the like; and an oil phase containing a hydrocarbon dispersant, and if necessary, a surfactant and a polymer-based dispersant, and the like can be heated under stirring in a state where they are mixed to carry out reverse phase suspension polymerization in a water-in-oil system.

When performing the reverse phase suspension polymerization, a monomer aqueous solution which contains ethylenically unsaturated monomers is dispersed in a hydrocarbon dispersion medium in the presence of a surfactant (and if necessary, a polymer-based dispersant). In this case, a timing of adding the surfactant, the polymer-based dispersant, or the like before the start of the polymerization reaction may be either before or after the addition of the monomer aqueous solution.

The polymerization may be carried out after dispersing the monomer aqueous solution in the hydrocarbon dispersion medium in which the polymer-based dispersant has been dispersed, and then further dispersing the surfactant in the hydrocarbon dispersion medium, from the viewpoint that an amount of the hydrocarbon dispersion medium remaining in the obtained water-absorbent resin can then be easily reduced.

The reverse phase suspension polymerization can be carried out in one stage or in multiple stages of two or more stages. The reverse phase suspension polymerization may be carried out in two or three stages from the viewpoint of increasing productivity.

In a case where reverse phase suspension polymerization is carried out in multiple stages of two or more stages, it is sufficient for stages after a second stage of reverse phase suspension polymerization to be carried out in the same manner as in a first stage of reverse phase suspension polymerization by adding ethylenically unsaturated monomers to a reaction mixture obtained in the first stage of polymerization reaction and mixing them, after performing the first stage of reverse phase suspension polymerization. In reverse phase suspension polymerization in each stage after the second stage, reverse phase suspension polymerization may be carried out by adding, in addition to ethylenically unsaturated monomers, the above-mentioned radical polymerization initiator within a range of molar ratios of the respective components to the ethylenically unsaturated monomers, based on an amount of ethylenically unsaturated monomers added during reverse phase suspension polymerization in each stage after the second stage. If necessary, the internal crosslinking agent may be used in reverse phase suspension polymerization in each stage after the second stage. In a case where the internal crosslinking agent is used, reverse phase suspension polymerization may be carried out by adding the internal crosslinking agent within a range of molar ratios of the respective components to the ethylenically unsaturated monomers based on an amount of ethylenically unsaturated monomers provided in each stage.

A temperature of the polymerization reaction varies depending on radical polymerization initiators used. A temperature of the polymerization reaction may be 20°C to 150°C, or 40°C to 120°C, from the viewpoint that the polymerization is then promptly performed, which shortens a polymerization time, and thereby economic efficiency increases, and that polymerization heat is then easily removed, and thereby the reaction is smoothly performed. A reaction time is generally 0.5 to 4 hours. Completion of the polymerization reaction can be confirmed from, for example, stop of temperature rising in the reaction system. Accordingly, a polymer of ethylenically unsaturated monomers is generally obtained in a state of a hydrogel polymer.

After the polymerization, post-polymerization crosslinking may be carried out by adding a crosslinking agent to the obtained hydrogel-like polymer and heating them. By performing the post-polymerization crosslinking, a degree of crosslinking of the hydrogel-like polymer can be increased, and thereby water-absorbent characteristics of the water-absorbent resin particles can be further improved.

Examples of crosslinking agents for performing the post-polymerization crosslinking include polyols such as ethylene glycol, propylene glycol, 1,4-butanediol, trimethylolpropane, glycerin, polyoxyethylene glycol, polyoxypropylene glycol, and polyglycerin; compounds having two or more epoxy groups, such as (poly)ethylene glycol diglycidyl ether, (poly)propylene glycol diglycidyl ether, and (poly)glycerin diglycidyl ether; haloepoxy compounds such as epichlorohydrin, epibromohydrin, and $\alpha$-methyl epichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. The crosslinking agent for the post-polymerization crosslinking may include polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether. These crosslinking agents may be used alone or in combination of two or more kinds thereof.

An amount of the crosslinking agent used in the post-polymerization crosslinking may be 0 to 0.03 moles, 0 to 0.01 moles, or 0.00001 to 0.005 moles in a molar ratio, per 1 mole of the ethylenically unsaturated monomer, from the viewpoint of exhibiting suitable water-absorbent characteristics by appropriately crosslinking the obtained hydrogel-like polymer.

It is sufficient for a timing for adding the crosslinking agent for the post-polymerization crosslinking to be after polymerization of ethylenically unsaturated monomers used for the polymerization. In a case of multi-stage polymerization, the crosslinking agent may be added after the multi-stage polymerization. From the viewpoint of a water content (to be described later), the crosslinking agent for the post-polymerization crosslinking may be added within a region of [water content immediately after polymerization $\pm$ 3% by mass], in consideration of heat generation during and after polymerization, retention due to process delay, system opening when a crosslinking agent is added, and fluctuation in moisture content due to addition of water associated with addition of a crosslinking agent.

Subsequently, drying is performed to remove water from the obtained hydrogel polymer. By drying, polymer particles containing the polymer of ethylenically unsaturated monomers are obtained. Examples of drying methods include a method (a) in which a hydrogel polymer in a state of being dispersed in a hydrocarbon dispersion medium is subjected to azeotropic distillation by heating from the outside, and the hydrocarbon dispersion medium is refluxed to remove water; a method (b) in which a hydrogel polymer is taken out by decantation and dried under reduced pressure; and a method (c) in which a hydrogel polymer is separated by filtration with a filter and dried under reduced pressure. The method (a) may be used for its simplicity in a production process.

It is possible to adjust a particle size of the water-absorbent resin particles by adjusting a rotation speed of a stirrer during the polymerization reaction or by adding a flocculating agent to the system after the polymerization reaction or at an initial time of drying. A particle size of the obtained water-absorbent resin particle can be increased by adding the flocculating agent. As the flocculating agent, an inorganic flocculating agent can be used. Examples of inorganic flocculating agents (for example, powdery inorganic flocculating agents) include silica, zeolite, bentonite, aluminum oxide, talc, titanium dioxide, kaolin, clay, and hydrotalcite. The flocculating agent may include at least one selected from the group consisting of silica, aluminum oxide, talc, and kaolin from the viewpoint of an excellent flocculation effect.

In the reverse phase suspension polymerization, the flocculating agent may be added by a method in which the flocculating agent is dispersed in a hydrocarbon dispersion medium of the same kind as that used in the polymerization, or water in advance, and then the mixture is mixed into a hydrocarbon dispersion medium containing a hydrogel polymer under stirring.

An amount of the flocculating agent added may be 0.001 to 1 part by mass, 0.005 to 0.5 parts by mass, or 0.01 to 0.2 parts by mass, with respect to 100 parts by mass of ethylenically unsaturated monomers used in the polymerization. By setting an amount of the flocculating agent added to be within the above range, it is easy to obtain water-absorbent resin particles having a desired particle size distribution.

The polymerization reaction can be carried out using various stirrers having a stirring blade. As the stirring blade, it is possible to use a flat plate blade, a lattice blade, a paddle blade, a propeller blade, an anchor blade, a turbine blade, a Pfaudler blade, a ribbon blade, a full zone blade, a max blend blade, and the like. A flat plate blade has a shaft (stirring shaft) and a flat plate portion (stirring portion) disposed around the shaft. The flat plate portion may have a slit or the like. When a flat plate blade is used as the stirring blade, uniformity of crosslinking of a polymer in the formed polymer particles tends to become high. When the uniformity of crosslinking is high, a local crosslinking density in the water-absorbent resin particles is small, and there are few softened portions. Therefore, it is easy to control a gel outflow proportion to be low while maintaining water-absorbent characteristics such as a water retention amount.

In the production of the water-absorbent resin particles, a surface portion of the hydrogel polymer may be crosslinked (surface-crosslinked) using a crosslinking agent in the drying step or any of subsequent steps. By performing surface crosslinking, the water-absorbent characteristics of the water-absorbent resin particles is easily controlled. The surface crosslinking may be performed at a timing when the hydrogel polymer has a specific water content. A timing of the surface crosslinking may be a time point at which a water content of the hydrogel polymer is 5% to 50% by mass, a time point at which a water content thereof is 10% to 40% by mass, or a time point at which a water content thereof is 15% to 35% by mass. A water content (% by mass) of the hydrogel polymer is calculated by the following formula.

$$\text{Water content} = [\text{Ww}/(\text{Ww} + \text{Ws})] \times 100$$

Ww: A water amount of a hydrogel polymer obtained by adding a water amount used, as desired, upon mixing a flocculating agent, a surface crosslinking agent, and the like to an amount obtained by subtracting a water amount extracted to the outside of the system by the drying step from a water amount contained in a monomer aqueous solution before polymerization in the all polymerization steps.

Ws: A solid fraction calculated from an amount of materials introduced, such as ethylenically unsaturated monomers, a crosslinking agent, and an initiator, each of which constitutes the hydrogel polymer.

Examples of crosslinking agents (surface crosslinking agents) for performing surface crosslinking include compounds having two or more reactive functional groups. Examples of crosslinking agents include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and $\alpha$-methyl epichlorohydrin; isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; hydroxyalkylamide compounds such as bis[N,N-di($\beta$-hydroxyethyl)]adipamide; and the like. The crosslinking agent may be used alone or in combination of two or more kinds thereof. The crosslinking agent may include a polyglycidyl compound, or may include at least one selected from the group consisting of (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether.

In general, a use amount of the surface crosslinking agent may be 0.00001 to 0.02 moles, 0.00005 to 0.01 moles, or 0.0001 to 0.005 moles, with respect to 1 mole of the ethylenically unsaturated monomer used in the polymerization, from the viewpoint of exhibiting suitable water-absorbent characteristics by appropriately crosslinking the obtained hydrogel-like polymer. In a case where a use amount of the surface crosslinking agent is 0.00001 moles or more, a crosslinking density in a surface portion of the water-absorbent resin particles is sufficiently increased, and thereby gel strength of the water-absorbent resin particles is easily increased. In a case where a use amount of the surface crosslinking agent is 0.02 moles or less, a water absorption amount of the water-absorbent resin particles is easily increased.

It is possible to obtain polymer particles, which are a dried product of the surface-crosslinked water-absorbent resin particles, by distilling off water and the hydrocarbon dispersion medium by a known method after the surface crosslinking.

The water-absorbent resin particles according to the present embodiment may be composed of only the polymer particles, but they can further contain, for example, various additional components selected from gel stabilizers, metal chelating agents, and flowability improvers (lubricants), and the like. The additional components may be disposed inside the polymer particles, on a surface of the polymer particles, or both of the inside and on the surface thereof. The additional component may be flowability improvers (lubricants). A flowability improver may be inorganic particles. Examples of inorganic particles include silica particles such as amorphous silica.

The water-absorbent resin particles may contain a plurality of inorganic particles disposed on the surface of the polymer. The inorganic particles can be disposed on the surface of the polymer particles by, for example, mixing the polymer particles and the inorganic particles. These inorganic particles may be silica particles such as amorphous silica. In a case where the water-absorbent resin particles contain inorganic particles disposed on the surface of the polymer particles, a ratio of the inorganic particles to a mass of the polymer particles may be 0.2% by mass or more, 0.5% by mass or more, 1.0% by mass or more, or 1.5% by mass or more, and it may be 5.0% by mass or less or 3.5% by mass or less. The inorganic particles referred to herein generally have a minute size as compared with a size of the polymer particles. For example, an average particle size of the inorganic particles may be 0.1 to 50 $\mu$m, 0.5 to 30 $\mu$m, or 1 to 20 $\mu$m. The average particle size referred to herein can be a value measured by a dynamic light scattering method or a laser diffraction/scattering method.

An absorber according to one embodiment contains the water-absorbent resin particles according to the present embodiment. The absorber according to the present embodiment can contain a fibrous substance, and it is, for example, a mixture containing the water-absorbent resin particles and the fibrous substance. The configuration of the absorber may be, for example, a configuration in which water-absorbent resin particles and the fibrous substances are uniformly mixed, a configuration in which water-absorbent resin particles are held between fibrous substances formed in a sheet shape or a layer shape, or another configuration.

Examples of fibrous substances include finely pulverized wood pulp; cotton; cotton linter; rayon; cellulose-based fibers such as cellulose acetate; synthetic fibers such as polyamides, polyesters, and polyolefins; a mixture of these fibers; and the like. The fibrous substance may be used alone or in combination of two or more kinds thereof. As the fibrous substance, hydrophilic fibers can be used.

A mass proportion of the water-absorbent resin particles in the absorber with respect to a total amount of the water-absorbent resin particles and the fibrous substance may be 2% to 100% by mass, 10% to 80% by mass, or 20% to 60% by mass.

Fibers may be adhered to each other by adding an adhesive binder to the fibrous substance in order to enhance shape retention properties before or during use of the absorber. Examples of adhesive binders include heat-sealing synthetic fibers, hot melt adhesives, and adhesive emulsions. The adhesive binder may be used alone or in combination of two or more kinds thereof.

Examples of heat-sealing synthetic fibers include full-melt binders such as polyethylene, polypropylene, and an ethylene-propylene copolymer; partial-melt binders formed of polypropylene and polyethylene in a side-by-side or core-and-sheath configuration; and the like. In the above-mentioned partial-melt binders, only a polyethylene portion can be thermal-bonded.

Examples of hot melt adhesives include a mixture of a base polymer such as an ethylene-vinyl acetate copolymer, a styrene-isoprene-styrene block copolymer, a styrene-butadiene-styrene block copolymer, a styrene-ethylene-butylene-styrene block copolymer, a styrene-ethylene-propylene-styrene block copolymer, and an amorphous polypropylene with a viscosity imparting agent, a plasticizer, an antioxidant, or the like.

Examples of adhesive emulsions include polymers of at least one monomer selected from the group consisting of methyl methacrylate, styrene, acrylonitrile, 2-ethylhexyl acrylate, butyl acrylate, butadiene, ethylene, and vinyl acetate.

The absorber according to the present embodiment may contain an inorganic powder (for example, amorphous silica), a deodorant, an antibacterial agent, a fragrance, and the like. In a case where the water-absorbent resin particles contain inorganic particles, the absorber may contain an inorganic powder in addition to the inorganic particles in the water-absorbent resin particles.

A shape of the absorber according to the present embodiment is not particularly limited, but it may be, for example, a sheet shape. A thickness of the absorber (for example, a thickness of a sheet-shaped absorber) may be, for example, 0.1 to 20 mm or 0.3 to 15 mm.

An absorbent article according to the present embodiment includes the absorber according to the present embodiment. Examples of other members constituting the absorbent article according to the present embodiment include a core wrap that retains the shape of the absorber; a liquid permeable sheet disposed on the outermost part on a side from which an absorption target liquid is infiltrated; a liquid impermeable sheet disposed on the outermost part on a side opposite to the side from which the absorption target liquid is infiltrated; and the like. Examples of the absorbent article include diapers (for example, paper diapers), toilet training pants, incontinence pads, hygiene products (sanitary napkins, tampons, and the like), sweat pads, pet sheets, portable toilet members, animal excrement treatment materials, and the like.

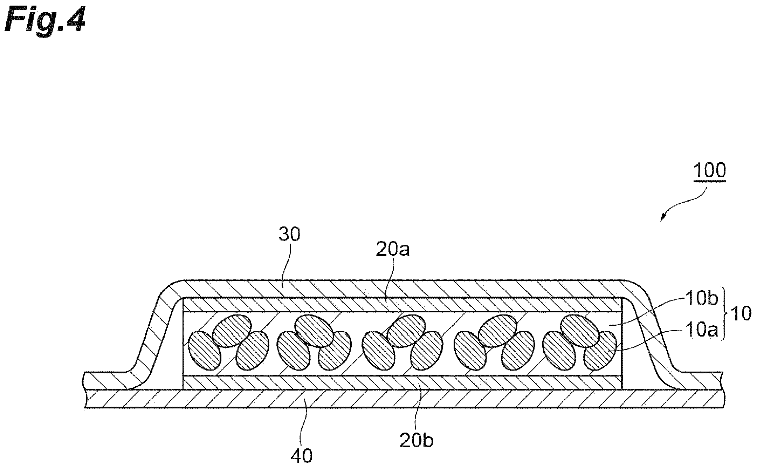

Fig. 4 is a cross-sectional view showing an example of an absorbent article. An absorbent article 100 shown in Fig. 4 includes an absorber 10, core wraps 20a and 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In Fig. 4, there is a portion shown to be a gap between the members, but the members may be in close contact with each other without the gap.

The absorber 10 has water-absorbent resin particles 10a according to the present embodiment and a fiber layer 10b containing a fibrous substance. The water-absorbent resin particles 10a are dispersed in the fiber layer 10b.

The core wrap 20a is disposed on one surface side of the absorber 10 (an upper side of the absorber 10 in Fig. 4) in a state of being in contact with the absorber 10. The core wrap 20b is disposed on the other surface side of the absorber 10 (a lower side of the absorber 10 in Fig. 4) in a state of being in contact with the absorber 10. The absorber 10 is disposed between the core wrap 20a and the core wrap 20b. Examples of the core wraps 20a and 20b include tissues, non-woven fabrics, and the like. The core wrap 20a and the core wrap 20b each have, for example, a main surface having the same size as that of the absorber 10.

The liquid permeable sheet 30 is disposed on the outermost part on a side from which an absorption target liquid is infiltrated. The liquid permeable sheet 30 is disposed on the core wrap 20a in a state of being in contact with the core wrap 20a. The liquid impermeable sheet 40 is disposed on the outermost part on a side opposite to the liquid permeable sheet 30, in the absorbent article 100. The liquid impermeable sheet 40 is disposed below the core wrap 20b in a state of being in contact with the core wrap 20b. The liquid permeable sheet 30 and the liquid impermeable sheet 40 each have, for example, a main surface wider than the main surface of the absorber 10, and outer edges of the liquid permeable sheet 30 and the liquid impermeable sheet 40 respectively extend around the absorber 10 and the core wraps 20a and 20b.

A magnitude relationship between the absorber 10, the core wraps 20a and 20b, the liquid permeable sheet 30, and the liquid impermeable sheet 40 is not particularly limited, and it is appropriately adjusted according to usage applications and the like of the absorbent article. A shape of the absorber 10 shown in Fig. 4 is maintained by being sandwiched between the two core wraps 20a and 20b. A method of maintaining a shape of the absorber by the core wrap is not limited thereto, and for example, the absorber may be sandwiched between one core wrap that has been folded. The core wrap may form a bag, and the absorber may be disposed inside the bag.

The liquid permeable sheet 30 may be a sheet formed of resin or fiber generally used in the technical field. From the viewpoint of liquid permeability, flexibility, and strength when the liquid permeable sheet 30 is used in an absorbent article, the liquid permeable sheet 30 may contain, for example, synthetic resins such as polyolefins such as polyethylene (PE) and polypropylene (PP); polyesters such as polyethylene terephthalate (PET), polytrimethylene terephthalate (PTT), and polyethylene naphthalate (PEN); polyamides such as nylon; and rayon, or synthetic fibers containing these synthetic resins, or the liquid permeable sheet 30 may be natural fibers including cotton, silk, hemp, or pulp (cellulose). The liquid permeable sheet 30 may contain synthetic fibers from the viewpoint of increasing strength of the liquid permeable sheet 30. Synthetic fibers may be particularly polyolefin fibers, polyester fibers, or a combination thereof. These materials may be used alone or in combination of two or more materials.

The liquid permeable sheet 30 may be a non-woven fabric, a porous sheet, or a combination thereof. Non-woven fabric is a sheet in which fibers are entwined instead of being woven. The non-woven fabric may be a non-woven fabric (short-fiber non-woven fabric) composed of short fibers (that is, staples), or may be a non-woven fabric (long-fiber non-woven fabric) composed of long fibers (that is, filaments). In general, staples may have a fiber length of several hundred millimeters or shorter, but its length is not limited thereto.

The liquid permeable sheet 30 may be thermal bonded non-woven fabrics, air through non-woven fabrics, resin bonded non-woven fabrics, spunbond non-woven fabrics, melt-blown non-woven fabrics, airlaid non-woven fabrics, spunlace non-woven fabrics, point-bonded non-woven fabrics, or a laminate of two or more of non-woven fabrics selected from these non-woven fabrics. These non-woven fabrics can be, for example, non-woven fabrics formed of the above-mentioned synthetic fibers or natural fibers. The laminate of two or more of non-woven fabrics may be, for example, a spunbond/melt-blown/spunbond non-woven fabric that is a composite non-woven fabric having spunbond non-woven fabric, melt-blown non-woven fabric, and spunbond non-woven fabric, which are laminated in this order. A thermal bonded non-woven fabric, an air through non-woven fabric, a spunbond non-woven fabric, or a spunbond/melt-blown/spunbond non-woven fabric may be used from the viewpoint of inhibiting liquid leakage.

A non-woven fabric used as the liquid permeable sheet 30 may have appropriate hydrophilicity from the viewpoint of liquid absorption performances of the absorbent article. From this viewpoint, the liquid permeable sheet 30 may be a non-woven fabric having a hydrophilicity of 5 to 200 as measured according to a measurement method of Pulp and Paper Test Method No. 68 (2000) by the Japan Technical Association of Pulp and Paper Industry. The hydrophilicity of the non-woven fabric may be 10 to 150. For details of Pulp and Paper Test Method No. 68, for example, WO2011/086843 can be referred to.

The hydrophilic non-woven fabric as described above may be formed of fibers, such as rayon fibers, showing appropriate hydrophilicity, or may be formed of fibers obtained by hydrophilizing hydrophobic chemical fibers such as polyolefin fibers and polyester fibers. Examples of methods of obtaining a non-woven fabric containing hydrophobic chemical fibers that have been hydrophilized include a method of obtaining a non-woven fabric by a spunbond technique using a mixture in which a hydrophilizing agent is added to hydrophobic chemical fibers, a method of using a hydrophilizing agent when producing a spunbond non-woven fabric from hydrophobic chemical fibers, and a method of impregnating a spunbond non-woven fabric obtained by using hydrophobic chemical fibers with a hydrophilizing agent. As the hydrophilizing agent, the following examples are used: anionic surfactants such as aliphatic sulfonic acid salts and higher alcohol sulfuric acid ester salts; cationic surfactants such as quaternary ammonium salts; nonionic surfactants such as polyethylene glycol fatty acid esters, polyglycerin fatty acid esters, and sorbitan fatty acid esters; silicone surfactants such as polyoxyalkylene-modified silicone; stain release agents formed of polyester-based, polyamide-based, acrylic-based, or urethane-based resin; and the like.

The liquid permeable sheet 30 may be a non-woven fabric that is moderately bulky and has a large fabric weight per unit area from the viewpoint of imparting favorable liquid permeability, flexibility, strength, and cushioning properties to an absorbent article, and from the viewpoint of accelerating a liquid permeation rate of an absorbent article. A fabric weight per unit area of the non-woven fabric used for the liquid permeable sheet 30 may be 5 to 200 g/m$^2$, 8 to 150 g/m$^2$, or 10 to 100 g/m$^2$. A thickness of the non-woven fabric used for the liquid permeable sheet 30 may be 20 to 1400 $\mu$m, 50 to 1200 $\mu$m, or 80 to 1000 $\mu$m.

The liquid impermeable sheet 40 is disposed on the outermost part on a side opposite to the liquid permeable sheet 30, in the absorbent article 100. The liquid impermeable sheet 40 is disposed below the core wrap 20b in a state of being in contact with the core wrap 20b. The liquid impermeable sheet 40 has, for example, a main surface wider than the main surface of the absorber 10, and an outer edge of the liquid impermeable sheet 40 extends around the absorber 10 and the core wraps 20a and 20b. The liquid impermeable sheet 40 prevents a liquid absorbed by the absorber 10 from leaking to the outside from the liquid impermeable sheet 40 side.

Examples of the liquid impermeable sheet 40 include sheets made of synthetic resins such as polyethylene, polypropylene, and polyvinyl chloride; sheets made of non-woven fabric such as a spunbond/melt-blown/spunbond (SMS) non-woven fabric in which a water-resistant melt blown non-woven fabric is sandwiched between high-strength spunbond non-woven fabrics; sheets made of a composite material of these synthetic resins and non-woven fabric (for example, spunbond non-woven fabric and spunlace non-woven fabric); and the like. The liquid impermeable sheet 40 may have breathability from the viewpoint that dampness generated when wearing the absorbent article is then reduced, and thereby discomfort to a wearer can be reduced. As the liquid impermeable sheet 40, it is possible to use a sheet made of a synthetic resin mainly composed of a low-density polyethylene (LDPE) resin. The liquid impermeable sheet 40 may be, for example, a sheet made of a synthetic resin and having a fabric weight per unit area of 10 to 50 g/m$^2$ from the viewpoint of ensuring flexibility so as not to impair a sensation of wearing the absorbent article.

The absorbent article 100 can be manufactured by, for example, a method including disposing the absorber 10 between the core wraps 20a, 20b, and disposing them between the liquid permeable sheet 30 and the liquid impermeable sheet 40. A laminate, in which the liquid impermeable sheet 40, the core wrap 20b, the absorber 10, the core wrap 20a, and the liquid permeable sheet 30 are laminated in this order, is pressurized as necessary.

The absorber 10 is formed by mixing the water-absorbent resin particles 10a with the fibrous substance. The absorber 10 may be formed by sorting out and selectively using the water-absorbent resin particles in which the above-described gel outflow proportion is 0% to 20%.

The gel outflow proportion of the water-absorbent resin particles 10a can be used as an index for increasing a permeation rate of physiological saline into the absorber 10 which contains the water-absorbent resin particles 10a and to which a load has been applied in a state where the absorber 10 absorbed water. A permeation rate of physiological saline into the absorber 10, to which a load has been applied in a state where the absorber 10 absorbed water, is increased by a method including reducing the gel outflow proportion of the water-absorbent resin particles 10a. For example, as the water-absorbent resin particles 10a, water-absorbent resin particles in which a gel outflow proportion is a predetermined value (for example, 20%) or less may be sorted out. Alternatively, the gel outflow proportion of the water-absorbent resin particles can be set to a predetermined value (for example, 20%) or less by selecting production conditions for the water-absorbent resin particles so that uniformity of crosslinking in the water-absorbent resin particles is high. Examples of methods for improving uniformity of crosslinking in the water-absorbent resin particles include using a flat plate blade as a stirring blade during a polymerization reaction, selecting a rotation speed of stirring within an appropriate range, adopting conditions in which heat is easily provided and removed (for example, a reaction in a hydrocarbon dispersion medium), using a crosslinking agent having an appropriately high reactivity, and the like.

According to the present embodiment, it is possible to provide a method of absorbing a liquid using the water-absorbent resin particles, absorber, or absorbent article according to the present embodiment. The method of absorbing a liquid according to the present embodiment includes a step of bringing an absorption target liquid into contact with the water-absorbent resin particles, absorber, or absorbent article according to the present embodiment.

EXAMPLES

Hereinafter, the present invention will be more specifically described with reference to examples. However, the present invention is not limited to these examples.

1. Production of water-absorbent resin particles

Example 1

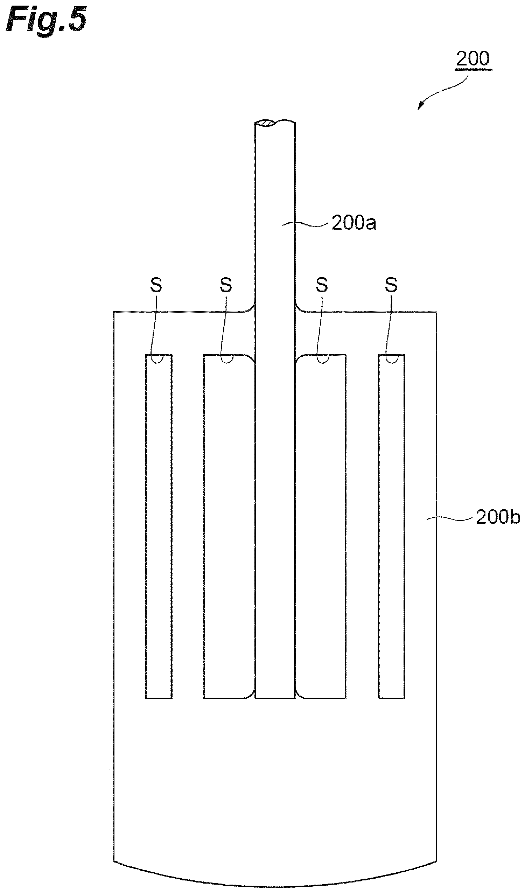

A cylindrical round-bottomed separable flask with the inner diameter of 11 cm and the internal volume of 2 L equipped with a reflux condenser, a dropping funnel, a nitrogen gas introduction tube, and a stirrer was prepared. The stirrer was equipped with a stirring blade 200 of which an outline is shown in Fig. 5. The stirring blade 200 includes a shaft 200a and a flat plate portion 200b. The flat plate portion 200b is welded to the shaft 200a and also has a curved distal end. The flat plate portion 200b is formed with four slits S extending along an axial direction of the shaft 200a. The four slits S are arranged in a width direction of the flat plate portion 200b, where the width of the two slits S at the inner side is 1 cm, and the width of the two slits S at the outer side is 0.5 cm. The length of the flat plate portion 200b is about 10 cm, and the width of the flat plate portion 200b is about 6 cm. 293 g of n-heptane, and 0.736 g of a dispersant (maleic anhydride-modified ethylene-propylene copolymer, HI-WAX 1105A, manufactured by Mitsui Chemicals, Inc.) were mixed in a prepared separable flask. The dispersant was dissolved in n-heptane by heating the mixture in the separable flask to 80°C while stirring with the stirrer. The formed solution was cooled to 50°C.

92.0 g (1.03 moles) of an aqueous solution of 80.5% by mass acrylic acid was put into a beaker with an internal capacity of 300 mL. 147.7 g of an aqueous solution of 20.9% by mass sodium hydroxide was added dropwise into the aqueous solution of acrylic acid in the beaker while cooling the beaker from the outside, and thereby 75 mol% of acrylic acid was neutralized. Next, 0.092 g of hydroxyethyl cellulose (HEC AW-15F, manufactured by Sumitomo Seika Chemicals Co., Ltd.), 0.092 g (0.339 mmol) of 2,2'-azobis(2-amidinopropane) dihydrochloride and 0.018 g (0.067 mmol) of potassium persulfate as a water-soluble radical polymerization initiator, and 0.0046 g (0.026 mmol) of ethylene glycol diglycidyl ether as an internal crosslinking agent were added and dissolved in the aqueous solution. Thereby, a first stage monomer aqueous solution was prepared.

The obtained monomer aqueous solution was put into a separable flask into which a solution of the dispersant was put, and the reaction solution in the separable flask was stirred for 10 minutes. A surfactant solution, in which 0.736 g of sucrose stearic acid ester (Mitsubishi-Chemical Foods Corporation, RYOTO Sugar Ester S-370, HLB value: 3) was dissolved in 6.62 g of n-heptane, was added into the reaction solution. The inside of the system was sufficiently replaced with nitrogen while stirring the reaction solution with the stirrer at a rotation speed of 425 rpm. A first stage polymerization reaction was caused to proceed by heating the separable flask in a warm water bath at 70°C for 60 minutes while continuing stirring, and thereby a first stage polymerization slurry solution containing a hydrogel-like polymer was obtained.

128.8 g (1.44 moles) of an aqueous solution of 80.5% by mass acrylic acid was put into another beaker with an internal capacity of 500 mL. 159.0 g of an aqueous solution of 27% by mass sodium hydroxide was added dropwise into the aqueous solution of acrylic acid in the beaker while cooling the beaker from the outside, and thereby 75 mol% of acrylic acid was neutralized. Next, 0.129 g (0.476 mmol) of 2,2'-azobis(2-amidinopropane) dihydrochloride and 0.026 g (0.096 mmol) of potassium persulfate as a water-soluble radical polymerization initiator, and 0.0117 g (0.067 mmol) of ethylene glycol diglycidyl ether as an internal crosslinking agent were added and dissolved in the aqueous solution. Thereby, a second stage monomer aqueous solution was prepared.

The first stage polymerization slurry solution was cooled to 25°C while stirring at a rotation speed of 650 rpm of the stirrer. A total amount of the second stage monomer aqueous solution was added thereinto. After replacing the inside of the system with nitrogen for 30 minutes, a second stage polymerization reaction was caused to proceed by heating the separable flask again in a warm water bath at 70°C for 60 minutes while continuing stirring.

Under stirring, 0.589 g of an aqueous solution of 45% by mass diethylenetriaminepentaacetic acid pentasodium was added into the reaction solution obtained after the second stage polymerization reaction. A temperature of the reaction solution was raised in an oil bath at 125°C, and 224.3 g of water was extracted out of the system by azeotropic distillation. Thereafter, 4.42 g (0.507 mmol) of an aqueous solution of 2% by mass ethylene glycol diglycidyl ether was added as a surface crosslinking agent, and by maintaining 83°C for 2 hours, a surface crosslinking reaction was caused to proceed.

The reaction solution was heated to 125°C to evaporate n-heptane, and thereby a dried product of polymer particles was obtained. This dried product was passed through a sieve having an opening of 850 $\mu$m, and 0.2% by mass of amorphous silica (Oriental Silicas Corporation, Tokusil NP-S) with respect to a mass of the polymer particles was mixed with the polymer particles. Thereby, 231.0 g of water-absorbent resin particles was obtained. The median particle size of the water-absorbent resin particles was 342 $\mu$m.

Example 2

233.0 g of water-absorbent resin particles, which contained 0.2% by mass of amorphous silica with respect to the mass of the polymer particles, was obtained in the same procedure as in Example 1 except that an amount of water extracted out of the reaction solution obtained after the second stage polymerization reaction by azeotropic distillation was 229.2 g. The median particle size of the water-absorbent resin particles was 368 $\mu$m.

Example 3

A first stage polymerization slurry solution was obtained in the same procedure as in Example 1 except that a first stage monomer aqueous solution was prepared by using only 0.0736 g (0.272 mmol) of potassium persulfate was used as a water-soluble radical polymerization initiator, and changing an amount of ethylene glycol diglycidyl ether to 0.010 g (0.057 mmol). A second stage monomer aqueous solution was prepared in the same procedure as in Example 1 except that only 0.090 g (0.333 mmol) of potassium persulfate was used as a water-soluble radical polymerization initiator.

221.5 g of water-absorbent resin particles, which contained 0.2% by mass of amorphous silica with respect to the mass of the polymer particles, was obtained in the same procedure as in Example 1 except that the obtained first stage polymerization slurry solution and second stage monomer aqueous solution were used, and an amount of water extracted out of the reaction solution obtained after the second stage polymerization reaction by azeotropic distillation was 264.3 g. The median particle size of the water-absorbent resin particles was 376 $\mu$m.

Example 4

A first stage polymerization slurry solution was obtained in the same procedure as in Example 1 except that a first stage monomer aqueous solution was prepared by using only 0.0736 g (0.272 mmol) of potassium persulfate was used as a water-soluble radical polymerization initiator, and changing an amount of ethylene glycol diglycidyl ether to 0.010 g (0.057 mmol). A second stage monomer aqueous solution was prepared in the same procedure as in Example 1 except that only 0.090 g (0.333 mmol) of potassium persulfate was used as a water-soluble radical polymerization initiator.

229.8 g of water-absorbent resin particles, which contained 0.2% by mass of amorphous silica with respect to the mass of the polymer particles, was obtained in the same procedure as in Example 1 except that the obtained first stage polymerization slurry solution and second stage monomer aqueous solution were used, and an amount of water extracted out of the reaction solution obtained after the second stage polymerization reaction by azeotropic distillation was 257.6 g. A median particle size of the water-absorbent resin particles was 362 $\mu$m.

Example 5

A cylindrical round-bottomed separable flask was prepared, which had four side wall baffles (baffle width: 7 mm), had an inner diameter of 11 cm and an internal volume of 2 L, and was equipped with a reflux condenser, a dropping funnel, a nitrogen gas introduction tube, and a stirrer. A stirrer including a stirring blade having four inclined paddle blades, which were surface-treated with a fluororesin and each of which has a blade diameter of 5 cm, in a two-tier manner was used. 451.4 g of n-heptane and 1.288 g of sorbitan monolaurate (NONION LP-20R, HLB value: 8.6, manufactured by NOF CORPORATION) were mixed in the prepared separable flask. The mixture in the separable flask was heated to 50°C while stirring with the stirrer to dissolve the sorbitan monolaurate in the n-heptane. The formed solution was cooled to 40°C.

92.0 g (1.03 moles) of an aqueous solution of 80.5% by mass acrylic acid was put into a beaker with an internal capacity of 300 mL. 147.7 g of an aqueous solution of 20.9% by mass sodium hydroxide was added dropwise into the aqueous solution of acrylic acid in the beaker while cooling the beaker from the outside, and thereby 75 mol% of acrylic acid was neutralized. Next, 0.1012 g (0.374 mmol) of potassium persulfate was added as a water-soluble radical polymerization initiator and dissolved in the aqueous solution to prepare a monomer aqueous solution.

The obtained monomer aqueous solution was added into the solution containing sorbitan monolaurate in the above-mentioned separable flask, and the inside of the system was sufficiently replaced with nitrogen. Thereafter, a polymerization reaction was caused to proceed by heating the separable flask in a warm water bath at 70°C for 60 minutes while stirring the reaction solution at a rotation speed of 700 rpm of the stirrer. By the polymerization reaction, a hydrogel-like polymer dispersed in the reaction solution was produced.

A dispersion liquid, which was obtained by dispersing 0.092 g of amorphous silica (Oriental Silicas Corporation, Tokusil NP-S) in 100 g of n-heptane in advance, was added into the reaction solution containing the hydrogel-like polymer while stirring at a rotation speed of 1,000 rpm of the stirrer. After stirring for 10 minutes, the separable flask was immersed in an oil bath at 125°C, and 91.1 g of water was extracted out of the system by azeotropic distillation. Thereafter, 4.14 g of an aqueous solution of 2% by mass ethylene glycol diglycidyl ether (ethylene glycol diglycidyl ether: 0.475 mmol) was added as a surface crosslinking agent, and the internal temperature was maintained at 83°C $\pm$ 2°C for 2 hours.

Water and n-heptane were evaporated by heating the reaction solution to 120°C until almost no evaporation substance was distilled off, and thereby a dried product of the polymer particles was obtained. This dried product was passed through a sieve having the opening of 850 $\mu$m to obtain 91.3 g of water-absorbent resin particles. A median particle size of the water-absorbent resin particles was 360 $\mu$m.

Comparative Example 1

90.1 of water-absorbent resin particles was obtained in the same procedure as in Example 5 except that an amount of water extracted out of the reaction solution by azeotropic distillation was 129.0 g. A median particle size of the water-absorbent resin particles was 358 $\mu$m.

Comparative Example 2

A cylindrical round-bottomed separable flask was prepared, which had four side wall baffles (baffle width: 7 mm), had an inner diameter of 11 cm and an internal volume of 2 L, and was equipped with a reflux condenser, a dropping funnel, a nitrogen gas introduction tube, and a stirrer. A stirrer including a stirring blade having four inclined paddle blades, which were surface-treated with a fluororesin and each of which has a blade diameter of 5 cm, in a two-tier manner was used. 293 g of n-heptane, and 0.736 g of a dispersant (maleic anhydride-modified ethylene-propylene copolymer, HI-WAX 1105A, manufactured by Mitsui Chemicals, Inc.) were mixed in a prepared separable flask. The dispersant was dissolved in n-heptane by heating the mixture in the separable flask to 80°C while stirring with the stirrer. The formed solution was cooled to 50°C.

92.0 g (1.03 moles) of an aqueous solution of 80.5% by mass acrylic acid was put into a beaker with an internal capacity of 300 mL. 147.7 g of an aqueous solution of 20.9% by mass sodium hydroxide was added dropwise into the aqueous solution of acrylic acid in the beaker while cooling the beaker from the outside, and thereby 75 mol% of acrylic acid was neutralized. Then, 0.092 g of hydroxyethyl cellulose (HEC AW-15F, manufactured by Sumitomo Seika Chemicals Co., Ltd.), 0.0736 g (0.272 mmol) of potassium persulfate as a water-soluble radical polymerization initiator, and 0.010 g (0.057 mmol) of ethylene glycol diglycidyl ether as an internal crosslinking agent were added and dissolved in the aqueous solution. Thereby, a first stage monomer aqueous solution was prepared.

The obtained monomer aqueous solution was put into a separable flask into which a solution of the dispersant was put, and the reaction solution in the separable flask was stirred for 10 minutes. A surfactant solution, in which 0.736 g of sucrose stearic acid ester (Mitsubishi-Chemical Foods Corporation, RYOTO Sugar Ester S-370, HLB value: 3) was dissolved in 6.62 g of n-heptane, was added into the reaction solution. The inside of the system was sufficiently replaced with nitrogen while stirring the reaction solution with the stirrer at a rotation speed of 550 rpm. A first stage polymerization reaction was caused to proceed by heating the separable flask in a warm water bath at 70°C for 60 minutes while continuing stirring, and thereby a first stage polymerization slurry solution containing a hydrogel-like polymer was obtained.

128.8 g (1.44 moles) of an aqueous solution of 80.5% by mass acrylic acid was put into another beaker with an internal capacity of 500 mL. 159.0 g of an aqueous solution of 27% by mass sodium hydroxide was added dropwise into the aqueous solution of acrylic acid in the beaker while cooling the beaker from the outside, and thereby 75 mol% of acrylic acid was neutralized. Next, 0.090 g (0.333 mmol) of potassium persulfate as a water-soluble radical polymerization initiator, and 0.0117 g (0.067 mmol) of ethylene glycol diglycidyl ether as an internal crosslinking agent were added and dissolved in the aqueous solution. Thereby, a second stage monomer aqueous solution was prepared.

The first stage polymerization slurry solution was cooled to 25°C while stirring at a rotation speed of 1000 rpm of the stirrer. A total amount of the second stage monomer aqueous solution was added thereinto. After replacing the inside of the system with nitrogen for 30 minutes, a second stage polymerization reaction was caused to proceed by heating the separable flask again in a warm water bath at 70°C for 60 minutes while continuing stirring.

Under stirring, 0.265 g of an aqueous solution of 45% by mass diethylenetriaminepentaacetic acid pentasodium was added into the reaction solution obtained after the second stage polymerization reaction. A temperature of the reaction solution was raised in an oil bath at 125°C, and 278.9 g of water was extracted out of the system by azeotropic distillation. Thereafter, 4.42 g (0.507 mmol) of an aqueous solution of 2% by mass ethylene glycol diglycidyl ether as a surface crosslinking agent was added, and maintained at 83°C for 2 hours.

The reaction solution was heated to 125°C to evaporate n-heptane, and thereby a dried product of polymer particles was obtained. This dried product was passed through a sieve having an opening of 850 μm, and 0.2% by mass of amorphous silica (Oriental Silicas Corporation, Tokusil NP-S) with respect to a mass of the polymer particles was mixed with the polymer particles. Thereby, 230.8 g of water-absorbent resin particles was obtained. A median particle size of the water-absorbent resin particles was 377 μm.

Comparative Example 3

A first stage polymerization slurry solution and a second stage monomer aqueous solution were obtained in the same procedure as in Example 1 except that a stirrer including a stirring blade having four inclined paddle blades, which were surface-treated with a fluororesin and each of which has a blade diameter of 5 cm, in a two-tier manner was used. A second stage polymerization reaction was caused to proceed in the same procedure as in Example 1 except that a rotation speed was changed to 1000 rpm. After the second stage polymerization reaction, 229.6 g of water-absorbent resin particles containing 0.2% by mass of amorphous silica with respect to a mass of polymer particles was obtained in the same procedure as in Example 1. A median particle size of the water-absorbent resin particles was 355 μm.

2. Evaluation of water-absorbent resin particles

2-1. Gel outflow proportion

2 g of water-absorbent resin particles was added into 58 g of physiological saline at 25°C while stirring the physiological saline in a glass beaker with a stirrer tip (length 3.0 cm, diameter 8 mm) at 600 rpm. After confirming that the water-absorbent resin particles had swollen and vortices on a liquid surface had converged, stirring was stopped. Thereafter, the mixture was left to stand for 10 minutes, and a swollen gel (gel swollen 30 times), which was formed when 2 g of the water-absorbent resin particles absorbed 58 g of the physiological saline, was obtained. Using the obtained swollen gel, a gel outflow proportion of the water-absorbent resin particles was measured in an environment of 25°C according to the above-described method including the steps (1), (2), (3), (4), (5), (6), and (7) and shown in Figs. 1, 2, and 3.

2-2. Water retention amount

A cotton bag (cotton broadcloth No. 60, 100 mm in width × 200 mm in length) into which 2.0 g of the water-absorbent resin particles was put was placed in a beaker having a capacity of 500 mL. 500 g of an aqueous solution of 0.9% by mass sodium chloride (physiological saline) was poured into the cotton bag containing the water-absorbent resin particles at once so that lumps could not be produced. The upper part of the cotton bag was bound with a rubber band, and the cotton bag was left to stand for 30 minutes. Thereby, the water-absorbent resin particles were swollen. The cotton bag after an elapse of 30 minutes was dehydrated for 1 minute using a dehydrator (manufactured by KOKUSAN Co., Ltd., product number: H-122) which had been set at a centrifugal force of 167 G, and a mass Wa (g) of the dehydrated cotton bag containing the swollen gel was measured. By performing the same operation without addition of the water-absorbent resin particles, a mass Wb (g) of an empty cotton bag upon moisturizing was measured, and a water retention amount under no pressurization was calculated by the following formula.

$$\text{Water retention amount (g/g)} = [\text{Wa} - \text{Wb}]/2.0$$

2-3. Water absorption amount

500 g of physiological saline (aqueous solution of 0.9% by mass sodium chloride) was stirred in a beaker having an internal volume of 500 mL with a magnetic stirrer bar (8 mmφ × 30 mm, without a ring) at 600 rpm. While stirring the physiological saline, 2.0 g of the water-absorbent resin particles were added, and the water-absorbent resin particles were dispersed so that lumps were not generated. The mixture was left to stand for 60 minutes in a state where the physiological saline was stirred to cause the water-absorbent resin particles to be sufficiently swollen. Thereafter, the contents of the beaker containing the swollen gel were filtered using a standard sieve having an opening of 75 μm and

a mass Wc (g). The sieve on which the swollen gel was placed was left to stand for 30 minutes in an inclined state with an inclination angle of about 30 degrees with respect to the horizontal to separate and filter excess water. Amass Wd (g) of the sieve on which the swollen gel was placed was measured. A water absorption amount of the water-absorbent resin particles for physiological saline under no pressurization was obtained by the following formula.

$$\text{Water absorption amount (g/g)} = (Wd - Wc)/2.0$$

2-4. Water absorption amount under load

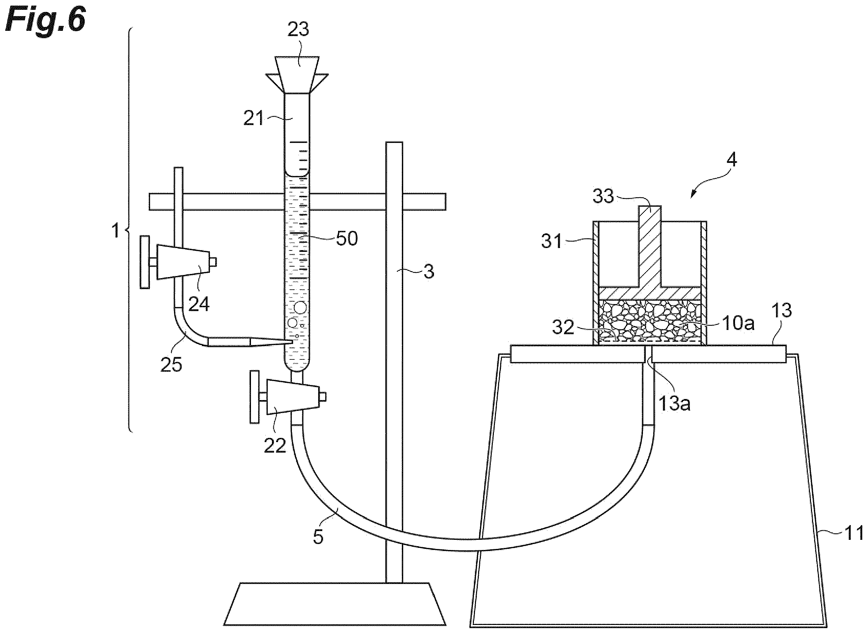

A water absorption amount of the water-absorbent resin particles for physiological saline under a load of 4.14 kPa (water absorption amount under a load) was measured using a device schematically shown in Fig. 6. A water absorption amount under a load was measured twice for one type of water-absorbent resin particles, and an average value of measurement values was obtained. The device of Fig. 6 includes a burette unit 1, a clamp 3, a conduit 5, a stand 11, a measurement table 13, and a measurement unit 4 placed on the measurement table 13. The burette unit 1 has a burette tube 21 on which a scale is engraved, a rubber stopper 23 for sealing an opening at an upper part of the burette tube 21, a cock 22 connected to a distal end of a lower part of the burette tube 21, an air introduction tube 25 connected to the lower part of the burette tube 21, and a cock 24. The burette unit 1 is fixed by the clamp 3. The flat plate-shaped measurement table 13 has a through hole 13a having a diameter of 2 mm and formed in the central portion of the measurement table 13, and is supported by the height-variable stand 11. The through hole 13a of the measurement table 13, and the cock 22 of the burette unit 1 are connected by the conduit 5. An inner diameter of the conduit 5 is 6 mm.
The measurement unit 4 has a cylinder 31 made of acrylic resin, a polyamide mesh 32 adhered to one opening of the cylinder 31, and a weight 33 that can move up and down in the cylinder 31. The cylinder 31 is placed on the measurement table 13 with the polyamide mesh 32 therebetween. An inner diameter of the cylinder 31 is 20 mm. An opening of the polyamide mesh 32 is 75 $\mu$m (200 mesh). The weight 33 has a diameter of 19 mm and a mass of 119.6 g, and can apply a load of 4.14 kPa to the water-absorbent resin particles 10a uniformly disposed on the polyamide mesh 32 as described later.
The measurement of a water absorption amount for physiological saline under a load of 4.14 kPa was performed in a room at 25°C by using the measurement device shown in Fig. 6. First, the cock 22 and the cock 24 of the burette unit 1 were closed, and 0.9% by mass physiological saline that had been adjusted to 25°C was put into the burette tube 21 through the opening at the upper part of the burette tube 21. Next, the opening on the upper part of the burette tube 21 was sealed with the rubber stopper 23, and then the cock 22 and the cock 24 were opened. The inside of the conduit 5 was filled with the 0.9% by mass saline solution 50 to prevent air bubbles from entering. A height of the measurement table 13 was adjusted so that a height of a water surface of the 0.9% by mass saline solution, which had reached the inside of the through hole 13a, was the same as a height of an upper surface of the measurement table 13. After the adjustment, the height of the water surface of the 0.9% by mass saline solution 50 in the burette tube 21 was read by the scale on the burette tube 21, and this position was defined as a zero point (value read at 0 seconds).
In the measurement unit 4, 0.10 g of the water-absorbent resin particles 10a was uniformly disposed on the polyamide mesh 32 in the cylinder 31, the weight 33 was disposed on the water-absorbent resin particles 10a, and the cylinder 31 was installed such that its center coincided with a conduit port at the center of the measurement table 13. An amount of decrease We (mL) in the physiological saline in the burette tube 21 60 minutes after the water-absorbent resin particles 10a started absorbing the physiological saline through the conduit 5 (that is, an amount of the physiological saline absorbed by the water-absorbent resin particles 10a) was read, and a water absorption amount of the water-absorbent resin particles 10a for the physiological saline under a load of 4.14 kPa was calculated by the following formula. The results are shown in Table 1.

$$\text{Water absorption amount under load (mL/g)} = \text{We (mL)/mass of}$$

$$\text{water-absorbent resin particles (g)}$$

2-5. Median particle size

The above-mentioned median particle size of the water-absorbent resin particles was measured by the following procedure. That is, JIS standard sieves were combined in the following order from the top: a sieve having an opening of 600 $\mu$m, a sieve having an opening of 500 $\mu$m, a sieve having an opening of 425 $\mu$m, a sieve having an opening of 300 $\mu$m, a sieve having an opening of 250 $\mu$m, a sieve having an opening of 180 $\mu$m, a sieve having an opening of 150 $\mu$m, and a saucer. 50 g of the water-absorbent resin particles was fed to the topmost sieve among the combination of the sieves, and using a Ro-Tap shaker (manufactured by Iida-seisakusho Japan Corporation), classification was performed according to JIS Z 8815 (1994). After the classification, a mass proportion of the particles remaining on each of the sieves was calculated as a mass percentage with respect to a total amount to determine a particle size distribution. By integrating proportions from weight percentages of particles remaining on the sieves in descending order of the particle sizes with regard to the particle size distribution, a relationship between the opening of the sieve and the integrated value of the proportions from the mass percentages of the particles remaining on the sieve was plotted on a log-probability paper. The plotted points on the probability paper were connected with straight lines, and a particle size corresponding to 50% by mass of the integrated mass percentage was obtained as a median particle size.

2-6. Evaluation of absorbent article

10 g of the water-absorbent resin particles and 6.7 g of pulverized pulp were uniformly mixed by air papermaking using an air flow type mixer (Padformer manufactured by OTEC Co., Ltd.), and thereby a sheet-shaped absorber having a size of 40 cm × 12 cm was produced. The absorber was sandwiched from its upper and lower sides with two sheets of tissue paper having a basis weight of 16 $g/m^2$ and having the same size as that of the absorber. The entirety was pressurized with a load of 196 kPa for 30 seconds. A polyethylenepolypropylene air-through type porous liquid permeable sheet, which has a basis weight of 22 $g/m^2$ and has the same size as that of the absorber, was placed on the tissue paper on the upper side. Accordingly, an absorbent article for evaluation having a configuration of a liquid permeable sheet/tissue paper/absorber/tissue paper was obtained.

In a room at a temperature of 25°C ± 2°C, the absorbent article for evaluation was placed on a horizontal table in a direction such that the liquid permeable sheet faced upward. A liquid injection cylinder, which includes a liquid storage part having a capacity of 100 mL and an injection tube having an inner diameter of 3 cm and extending from a bottom part of the liquid storage part, was prepared, and this liquid injection cylinder was maintained in a state where a distal end of the injection tube was in contact with the central portion of the absorbent article for evaluation. 80 mL of physiological saline, which was colored with a small amount of Blue No. 1 and adjusted to 25°C ± 1°C, was injected into the liquid storage part of the cylinder at once, and the physiological saline was caused to be absorbed by the absorber from the distal end of the injection tube. Using a stopwatch, a time until the physiological saline completely disappeared from the cylinder was measured. This time was defined as a first permeation time (seconds). At a time point after a lapse of 5 minutes from the injection of the physiological saline, a weight, which has a mass of 5 kg and has a square bottom surface of 10 cm × 10 cm, was placed on the central portion of the absorbent article for evaluation to apply a load on a portion of the absorbent article for evaluation in which the physiological saline was absorbed. After loading for 4 minutes, the weight was removed from the top of the absorbent article for evaluation. One minute after removing the weight (corresponding to 10 minutes from the first injection of the physiological saline), physiological saline colored with a small amount of Blue No. 1 was injected in the same method as in the first injection of physiological saline, and a second permeation time (seconds) was measured. At a time point after a lapse of 5 minutes from the second injection of the physiological saline, the weight was placed again on the absorbent article for evaluation, a load was applied to the absorbent article for evaluation for 4 minutes, and thereafter the weight was removed. One minute after removing the weight (corresponding to 20 minutes from the first injection of the physiological saline), physiological saline colored with a small amount of Blue No. 1 was injected in the same method as in the first injection of physiological saline, and a third permeation time was measured.

Table 1

| | Water-absorbent resin particles | | | | Permeation rate in absorbent article (sec.) | | |
|---|---|---|---|---|---|---|---|
| | Water retention amount [g/g] | Water absorption amount [g/g] | Water absorption amount under load [mL/g] | Gel outflow proportion [%] | First time | Second time | Third time |
| Ex. 1 | 40 | 56 | 28 | 4 | 36 | 69 | 117 |
| Ex. 2 | 45 | 62 | 21 | 4 | 35 | 69 | 108 |
| Ex. 3 | 45 | 63 | 14 | 16 | 35 | 72 | 111 |
| Ex. 4 | 42 | 60 | 18 | 19 | 35 | 66 | 112 |
| Comp. Ex. 1 | 34 | 64 | 17 | 25 | 43 | 109 | 126 |

(continued)

| | Water-absorbent resin particles | | | | Permeation rate in absorbent article (sec.) | | |
|---|---|---|---|---|---|---|---|
| | Water retention amount [g/g] | Water absorption amount [g/g] | Water absorption amount under load [mL/g] | Gel outflow proportion [%] | First time | Second time | Third time |
| Comp. Ex. 2 | 38 | 67 | 4 | 29 | 53 | 125 | 149 |
| Comp. Ex. 3 | 50 | 69 | 11 | 29 | 39 | 65 | 130 |
| Comp. Ex. 4 | 51 | 68 | 17 | 59 | 36 | 62 | 139 |

Based on the results shown in Table 1, it was confirmed that, in the water-absorbent resin particles of the examples in which a gel outflow proportion was 20% or less, a permeation rate (permeation rate of the third time) was significantly increased after a load was applied to the absorber that had absorbed water, as compared with the water-absorbent resin particles of the comparative examples.

Reference Signs List

1: burette unit, 3: clamp, 4: measurement unit, 5: conduit, 10: absorber, 10a: water-absorbent resin particle, 10b: fiber layer, 11: stand, 13: measurement table, 13a: through hole, 20a, 20b: core wrap, 21: burette tube, 22: cock, 23: rubber stopper, 24: cock, 25: air introduction tube, 30: liquid permeable sheet, 31: cylinder, 32: polyamide mesh, 33:

weight, 40: liquid impermeable sheet, 41: tube shaped body, 41A:
opening, 41B: bottom part, 42: disc, 45: swollen gel, 46: first weight, 47:
second weight, 100: absorbent article, 200: stirring blade, 200a: shaft,
200b: flat plate portion, H: through hole.

## Claims

1. Water-absorbent resin particles,
wherein a gel outflow proportion measured by a method including the following (1), (2), (3), (4), (5), (6), and (7) in this order in an environment of 25°C $\pm$ 2°C is 0% to 20%:

(1) fixing a tube shaped body of circular cross section with an inner diameter of 5.0 cm having a bottom part which blocks one end portion of the tube shaped body, the bottom part forming an opening with a diameter of 3.0 cm at a central portion, in a direction so that a longitudinal direction of the tube shaped body is vertical and the bottom part is at a downward side;
(2) disposing a disc with a diameter of 4.9 cm and a thickness of 3.0 mm on the bottom part in an inner side of the tube shaped body in a direction so that a main surface of the disc is horizontal, the disc forming a through hole at a central portion, the through hole having a wall surface inclined with respect to the main surface of the disc so that the through hole is narrowest at a central location in a thickness direction of the disc, a diameter of the through hole being 8.0 mm as a maximum value at locations of both main surfaces of the disc and being 5.0 mm as a minimum value at the central location in the thickness direction of the disc;
(3) putting 20 g of a swollen gel into the tube shaped body, thereby disposing the swollen gel on the disc, the swollen gel being formed by allowing the water-absorbent resin particles to absorb physiological saline, the swollen gel having a mass 30 times the mass of the water-absorbent resin particles before absorbing the physiological saline;
(4) compressing the swollen gel for 30 seconds by placing a column shaped first weight with a diameter of 4.9 mm on the swollen gel in the tube shaped body, the first weight having a mass of 500 g;
(5) further compressing the swollen gel under a load of 2000 g by placing a column shaped second weight with a diameter of 4.9 mm on the first weight, the second weight having a mass of 1500 g;
(6) measuring a mass W (g) of the swollen gel flowed out from the through hole of the disc while the swollen gel is compressed for 30 seconds under a load of 2000 g; and

(7) calculating the gel outflow proportion (%) by the following formula,

$$\text{gel outflow proportion (\%)} = (W/20) \times 100.$$

**2.** The water-absorbent resin particles according to claim 1, wherein a water absorption amount of the water-absorbent resin particles for physiological saline under a load of 4.14 kPa is 12 mL/g or more.

**3.** The water-absorbent resin particles according to claim 1 or 2, wherein a water retention amount of the water-absorbent resin particles for physiological saline is 40 to 46 g/g.

**4.** An absorbent article comprising:

a liquid impermeable sheet;
an absorber; and
a liquid permeable sheet,
wherein the liquid impermeable sheet, the absorber, and the liquid permeable sheet are disposed in this order, and
the absorber comprises the water-absorbent resin particles according to any one of claims 1 to 3.

**5.** A method for producing water-absorbent resin particles, the method comprising:
sorting out water-absorbent resin particles having a gel outflow proportion measured by a method including the following (1), (2), (3), (4), (5), (6), and (7) in this order in an environment of 25°C ± 2°C is 0% to 20%:

(1) fixing a tube shaped body of circular cross section with an inner diameter of 5.0 cm having a bottom part which blocks one end portion of the tube shaped body, the bottom part forming an opening with a diameter of 3.0 cm at a central portion, in a direction so that a longitudinal direction of the tube shaped body is vertical and the bottom part is at a downward side;
(2) disposing a disc with a diameter of 4.9 cm and a thickness of 3.0 mm on the bottom part in an inner side of the tube shaped body in a direction so that a main surface of the disc is horizontal, the disc forming a through hole at a central portion, the through hole having a wall surface inclined with respect to the main surface of the disc so that the through hole is narrowest at a central location in a thickness direction of the disc, a diameter of the through hole being 8.0 mm as a maximum value at locations of both main surfaces of the disc and being 5.0 mm as a minimum value at the central location in the thickness direction of the disc;
(3) putting 20 g of a swollen gel into the tube shaped body, thereby disposing the swollen gel on the disc, the swollen gel being formed by allowing the water-absorbent resin particles to absorb physiological saline, the swollen gel having a mass 30 times the mass of the water-absorbent resin particles before absorbing the physiological saline;
(4) compressing the swollen gel for 30 seconds by placing a column shaped first weight with a diameter of 4.9 mm on the swollen gel in the tube shaped body, the first weight having a mass of 500 g;
(5) further compressing the swollen gel under a load of 2000 g by placing a column shaped second weight with a diameter of 4.9 mm on the first weight, the second weight having a mass of 1500 g;
(6) measuring a mass W (g) of the swollen gel flowed out from the through hole of the disc while the swollen gel is compressed for 30 seconds under a load of 2000 g; and
(7) calculating the gel outflow proportion (%) by the following formula,

$$\text{gel outflow proportion (\%)} = (W/20) \times 100.$$

**6.** A method for increasing a permeation rate of physiological saline into an absorber comprising water-absorbent resin particles after the absorber is applied with a load in a state where the absorber has absorbed water, the method comprising:
reducing a gel outflow proportion of the water-absorbent resin particles measured by a method including the following (1), (2), (3), (4), (5), (6), and (7) in this order in an environment of 25°C ± 2°C:

(1) fixing a tube shaped body of circular cross section with an inner diameter of 5.0 cm having a bottom part which blocks one end portion of the tube shaped body, the bottom part forming an opening with a diameter of 3.0 cm at a central portion, in a direction so that a longitudinal direction of the tube shaped body is vertical and the bottom part is at a downward side;

(2) disposing a disc with a diameter of 4.9 cm and a thickness of 3.0 mm on the bottom part in an inner side of the tube shaped body in a direction so that a main surface of the disc is horizontal, the disc forming a through hole at a central portion, the through hole having a wall surface inclined with respect to the main surface of the disc so that the through hole is narrowest at a central location in a thickness direction of the disc, a diameter of the through hole being 8.0 mm as a maximum value at locations of both main surfaces of the disc and being 5.0 mm as a minimum value at the central location in the thickness direction of the disc;

(3) putting 20 g of a swollen gel into the tube shaped body, thereby disposing the swollen gel on the disc, the swollen gel being formed by allowing the water-absorbent resin particles to absorb physiological saline, the swollen gel having a mass 30 times the mass of the water-absorbent resin particles before absorbing the physiological saline;

(4) compressing the swollen gel for 30 seconds by placing a column shaped first weight with a diameter of 4.9 cm on the swollen gel in the tube shaped body, the first weight having a mass of 500 g;

(5) further compressing the swollen gel under a load of 2000 g by placing a column shaped second weight with a diameter of 4.9 cm on the first weight, the second weight having a mass of 1500 g;

(6) measuring a mass W (g) of the swollen gel flowed out from the through hole of the disc while the swollen gel is compressed for 30 seconds under a load of 2000 g; and

(7) calculating the gel outflow proportion (%) by the following formula,

$$\text{gel outflow proportion (\%)} = (W/20) \times 100.$$

## Fig.1

# Fig.2

# Fig.3

Fig.4

# *Fig.5*

**Fig.6**

## INTERNATIONAL SEARCH REPORT

| | International application No. |
|---|---|
| | PCT/JP2020/009468 |

**A. CLASSIFICATION OF SUBJECT MATTER**
Int.Cl. C08G59/42(2006.01)i, C08L101/14(2006.01)i, A61F13/53(2006.01)i
FI: C08G59/42, A61F13/53300, C08L101/14

According to International Patent Classification (IPC) or to both national classification and IPC

**B. FIELDS SEARCHED**

Minimum documentation searched (classification system followed by classification symbols)
Int.Cl. C08G59/42, C08L101/14, A61F13/53

Documentation searched other than minimum documentation to the extent that such documents are included in the fields searched
| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2018/180864 A1 (SUMITOMO SEIKA CHEMICALS CO., LTD.) 04.10.2018 (2018-10-04), claims 1-4, paragraphs [0060]-[0101], examples 1-4, comparative examples 1-3 | 1-4<br>5-6 |
| X<br>A | WO 2016/006135 A1 (SUMITOMO SEIKA CHEMICALS CO., LTD.) 14.01.2016 (2016-01-14), claims 1-6, paragraphs [0058]-[0117], examples 1-6, comparative examples 1-4 | 1-4<br>5-6 |
| X<br>A | JP 2017-155194 A (SUMITOMO SEIKA CHEMICALS CO., LTD.) 07.09.2017 (2017-09-07), claims 1-9, paragraphs [0064]-[0094], examples 1-6, comparative examples 1-4 | 1-4<br>5-6 |
| X<br>A | JP 2016-028131 A (SUMITOMO SEIKA CHEMICALS CO., LTD.) 25.02.2016 (2016-02-25), claims 1-6, paragraphs [0073]-[0134], examples 1-3, comparative examples 1, 2 | 1-4<br>5-6 |

☐ Further documents are listed in the continuation of Box C. ☒ See patent family annex.

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 15.05.2020 | 26.05.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

28

INTERNATIONAL SEARCH REPORT

Information on patent family members

International application No.

PCT/JP2020/009468

```
WO 2018/180864 A1  04.10.2018     CN 110325562 A
                                  KR 10-2019-0127706 A


WO 2016/006135 A1  14.01.2016     US 2016/0030919 A1
                                  claims 1-6, paragraphs [0082]-[0131]
                                  EP 2993189 A1
                                  EP 3357935 A1
                                  CN 104918964 A
                                  KR 10-2016-0017648 A
                                  CA 2953888 A1


JP 2017-155194 A   07.09.2017     (Family: none)


JP 2016-028131 A   25.02.2016     US 2017/0210831 A1
                                  claims 1-10, paragraphs [0084]-[0133]
                                  examples 1-3, comparative examples 1, 2
                                  EP 3153528 A1
                                  KR 10-2016-0142416 A
                                  CA 2954027 A1
                                  CN 106471013 A
```

**EP 3 936 549 A1**

## REFERENCES CITED IN THE DESCRIPTION

### Patent documents cited in the description

- JP 2006199805 A
- WO 2011086843 A